# EUROPEAN PATENT SPECIFICATION

(11) **EP 2 451 466 B1**
(45) Date of publication and mention of the grant of the patent: **30.03.2016**
(21) Application number: 10797691.2
(22) Date of filing: 03.07.2010
(51) Int. Cl.: A61K 35/16, G01N 33/92

(54) **APOLIPOPROTEIN CIII IN PRE- AND TYPE 2 DIABETES**
APOLIPOPROTEIN CIII BEI PRÄDIABETES UND DIABETES VOM TYP 2
APOLIPOPROTÉINE CIII DANS LE PRÉDIABÈTE ET LE DIABÈTE DE TYPE 2

(30) Priority: 07.07.2009 US 223502 P
(43) Date of publication of application: 16.05.2012
(73) Proprietor: Intrinsic Bioprobes, Inc., Tempe, AZ 85284 (US)
(72) Inventor: KIERNAN, Urban A., Chandler Arizona 85249 (US); NEDELKOV, Dobrin, Tempe Arizona 85284 (US)
(74) Representative: Hanna, Peter William Derek
(86) International application number: PCT/US2010/040998
(87) International publication number: WO 2011/005718

(56) References cited:
- WO-A1-2006/073195
- WO-A1-2006/110082
- WO-A1-2007/055341
- WO-A2-2007/112055
- US-A1- 2002 161 187
- US-A1- 2004 224 304
- US-A1- 2007 087 448
- US-A1- 2008 300 170
- JOVEN ET AL.: 'Concentrations of lipids and apolipoproteins in patients with clinically well-controlled insulin-dependent and non-insulin-dependent diabetes.' CLINICAL CHEMISTRY vol. 35, no. 5, 1989, pages 813 - 816, XP008150816
- HOSPATTANKAR ET AL: "Amino acid sequence of human plasma apolipoprotein C-III from normalipidemic subjects", FEBS LETTERS, vol. 197, no. 1-2, 3 March 1986 (1986-03-03), pages 67-73,

## Description

### FIELD OF THE INVENTION

This invention relates to methods for determining, diagnosing, and/or assessing pre-diabetes, type 2 diabetes, insulin resistance and their related conditions by detecting levels and modulations of Apolipoprotein CIII (1) (ApoCIII(1)). This invention also relates to methods for screening molecules that modulate ApoCIII (1) and its use in the treatment and/or amelioration of symptoms that are related to pre-diabetes, type 2 diabetes, insulin resistance and their related conditions.

### BACKGROUND OF THE INVENTION

Type 2 diabetes (also known as diabetes mellitus type 2, non-insulin-dependent diabetes mellitus or adult on-set diabetes) is a metabolic disorder that is characterized by high blood glucose. This condition is a result of either a lack of insulin produced by the body or the developed tolerance to insulin by the cells of the body (Insulin Resistance). Since insulin is essential for the absorption of glucose from the blood, perturbations in insulin homeostasis eventually result in loss of glycemic control and the development of the diabetic condition. If left uncontrolled, excessive blood glucose will eventually lead to a multitude of complications, including but not limited to: blindness, skin ulcerations, amputations, heart disease and kidney disease. Many proposed theories exist regarding the association of multiple complications that are linked to the loss of glycemic control, however, the mechanisms in which these multiple pathways interact is still unknown.

As of 2007, the Centers of Disease Control determined that 7.8% of the US population (23.6 million people) have type 2 diabetes and it is estimated that >20% of Americans are pre-diabetic. Due to the debilitating nature of these conditions and their associated cost of treatment, more effective and efficient methods for earlier detection are paramount to the health of the US populace and western civilization. The "gold standard" for evaluating the level of glycemic control is the Oral Glucose Tolerance Test (OGTT). This test involves measuring the fasting blood glucose of an individual or patient and then obtaining a blood glucose measurement 2 hours post administration of an oral glucose solution. Current guidelines define normal glucose tolerance (NGT) as a 2 hour glucose level ≤ 140 mg/dl. The criteria for type 2 diabetes (DM) is a 2 hour glucose of ≥ 200 mg/dl and the definition of impaired glucose tolerance (IGT or pre-diabetes) is between 140 and 200 mg/dl. Even though these criteria are sanctioned by both the American Diabetes Association and the World Health Organization, these values are not without clinical error or subject to interpretation.

US 2008/300170 A1 describes the identification of biological markers associated with an increased risk of developing Diabetes, as well as methods of using such biological markers in diagnosis and prognosis of Diabetes. The disclosed biological markers may indicate new targets for therapy or constitute new therapeutics for the treatment or prevention of Diabetes.

WO 2007/112055 A2 relates to a method for determining the concentration and modifications of apolipoprotein in biological samples including plasma, serum, and lipoprotein fractions. The method described is for use as a tool for diagnosing a number of diseases including diabetes, stroke, stress, Alzheimer's disease and cardiovascular diseases.

WO 2007/055341 A1 describes a method for diagnosing or preliminary diagnosing diabetes which is useful for the detection or prevention of diabetes and applicable also to a multimarker system and a kit for diagnosing or preliminary diagnosing diabetes. The diagnosis or preliminary diagnosis of diabetes is carried out by selecting as a marker substance for diabetes, one or more proteins selected from the group consisting of transthyretin, apolipoprotein CII, apolipoprotein CIII, serum albumin and related substances thereof in blood and comparing the concentration thereof with that of a normal value.

WO 2006/110082 A1 relates to a method and a kit for diagnosing or prognosing susceptibility to develop type 2 diabetes, the metabolic syndrome, sub clinical atherosclerosis, myocardial infarct, stroke or clinical manifestations of diabetes in a subject. The method comprises detecting and quantifying the amount of bound protein (s), (apoCIII, apoCI, apoAI or apoE) on small dense low density lipoproteins (sdLDL) particles in a blood sample from said subject.

WO 2006/073195 A1 describes a method for diagnosing or pre-diagnosing diabetes that is useful for detection or prevention of diabetes and applicable to a multi-marker system and a kit for diagnosing or pre-diagnosing diabetes. As a diabetes marker substance, one or more proteins selected from the group consisting of transthyretin, apolipoprotein CII, apolipoprotein CIII, serum albumin, and substances related to them in blood are selected, and the concentration of each substance is compared with a normal value and the diagnosis or pre-diagnosis of diabetes is conducted.

US 2002/161187 A1 describes the use of a combination of preparatory steps in conjunction with mass spectroscopy and time-of-flight detection procedures to maximize the diversity of biopolymers which are verifiable within a particular sample. The cohort of biopolymers verified within such a sample is then viewed with reference to their ability to evidence at least one particular disease state; thereby enabling a diagnostician to gain the ability to characterize either the presence or absence of said at least one disease state relative to recognition of the presence and/or the absence of said biopolymer. One such disease state is Type 2 diabetes.

US 2007/0087448 relates to methods to diagnose and follow the progression of disease through the use of protein analysis. It describes a method for utilizing mass spectrometry to analyze biological samples, particularly in connection with monitoring the health status or disease state of a subject. A biological sample containing a bodily fluid, such as blood, fractionated blood, plasma, fractionated plasma, serum, fractionated serum, urine or saliva is diluted and subjected to mass spectrometry, for example matrix-assisted laser desorption ionization time-of-flight (MALDI-TOF) mass spectrometry, to yield a plurality of mass spectrometry peaks, at least one of which is analyzed. The invention described includes a method for diagnosing, evaluating or monitoring the health of a subject. For example, the method can be used to detect the presence, absence or status of diabetes, pre-diabetes, or insulin resistance.
US 2004/0224304 A1 relates to methods of identifying candidate compounds for the treatment of type I diabetes by identifying those test compounds that inhibit an apoCIII-induced increase in intracellular calcium concentration in pancreatic beta cells. It describes methods for diagnosing Type I diabetes or a propensity to develop type I diabetes by comparing an amount of sialylated apoCIII in a blood serum sample from a subject with an amount of sialylated apoCIII in a control blood serum sample and diagnosing those subjects with an elevated amount of sialylated apoCIII in the blood serum sample relative to the control as having type I diabetes, or having a propensity to develop type I diabetes.

Joven et al., in the article entitled "Concentrations of lipids and apolipoproteins in patients with clinically well-controlled insulin-dependent and non-insulin dependent diabetes", (Clinical Chemistry, 1989, vol. 35(5), pp 813-816) describe a study where the triglyceride and cholesterol content of total, very-low-, intermediate-, low-, and high-density lipoproteins, and of apolipoproteins (Apo) AI, All, B, CII, CIII, and E were determined in plasma from 107 patients with clinically well-controlled diabetes and from 66 age- and weight-matched healthy normal subjects. Concentrations of Apo B, CIII, and E were higher in all diabetic patients than in the healthy controls, but those of apo CII did not differ statistically between diabetics and non-diabetics.

A recent development in diabetes care included the adoption of guidelines for the use of the protein biomarker hemoglobin A1C (HbA1C) in the diagnosis of diabetes and pre-diabetes. Due to the longevity of hemoglobin in the blood, it serves as a long term measure of glycemic control. Although long used as a biomarker of whether or not a patient's diabetes treatment is sufficient, conflicting data previously prevented the use of HbA1C in diabetes determination. Even with the acceptance of these new guidelines, and their use in conjunction with the standard OGTT criteria and fasting blood glucose levels, the diagnosis of pre-diabetes or type 2 diabetes still ultimately relies on the interpretation and the judgment of the diagnosing physician.
Due to the pandemic nature of type 2 diabetes, pre-diabetes, insulin resistance and the development of their associated complications, there is a world wide need for additional detection/diagnostic/assessment methods to allow for the earliest possible intervention and to provide a means to evaluate the effectiveness of treatment through life-style changes and/or medication. A need also exists for additional metabolic or endocrine targets for the development of treatments that alleviate or ameliorate the problems and symptoms associated with these related conditions.

### SUMMARY OF THE INVENTION

As shown here for the first time, modulations in the concentration of ApoCIII(1) correlate with the 2 hour glucose values obtained after the application of an OGTT and the clinical diagnosis of certain metabolic disease states. These findings identify ApoCIII(1) as a biomarker for the clinical assessment/diagnosis of type 2 diabetes, pre- diabetes, insulin resistance and their related conditions. It also stands to reason that ApoCIII(1) may also mechanistically participate in the initiation/development and pathology of these disease states and therefore may also have utility as a therapeutic target for the amelioration and/or treatment of the disease symptoms or condition.

One objective of the present invention is to provide a new target and screening method for the analysis of ApoCIII(1) in the in-vitro and in-vivo detection and/or diagnosis of type 2 diabetes, pre-diabetes, insulin resistance and their related conditions.
Another objective of the present invention is to use ApoCIII(1) as a new target and screening method for the in-vitro and in-vivo detection and/or diagnosis of type 2 diabetes, pre-diabetes, insulin resistance and their related conditions.
A further objective of the present invention is to use ApoCIII(1) as a new target for molecules that modulate ApoCIII(1) and/or in the screening of molecules aimed at the in-vitro and/or in-vivo modulation of its expression, activity and/or clearance in the treatment of type 2 diabetes, pre-diabetes, insulin resistance and their related conditions.
Yet a further objective of the present invention is to use ApoCIII(1) as a new target for molecules that modulate ApoCIII(1) and/or in the screening of molecules aimed at the in-vitro and/or in-vivo modulation of its expression, activity and/or clearance in the treatment of type 2 diabetes, pre-diabetes, insulin resistance and their related conditions.
Some novel features that are considered characteristic of the invention are set forth with particularity in the claims. The invention itself, however, both as to its structure and its operation together with the additional objectives and advantages thereof will best be understood from the following description of the exemplary embodiment of the present invention when read in conjunction with the accompanying drawings. Unless specifically noted, it is intended that the words and phrases in the specification and claims be given the ordinary and accustomed meaning for those of ordinary skill in the application of the art or arts. If any other meaning is intended, the specification will specifically state that special meaning is being applied to the word or phrase.

### BRIEF DESCRIPTION OF THE DRAWINGS

The object and features of the present invention will be better understood with reference to the following detailed description and drawings.
FIG. 1 illustrates representative mass spectral results of the analysis of ApoCIII and its isoforms and variants from human plasma samples using mass spectrometric immunoassay. The variants, ApoCIII(0) and ApoCIII(2) do not form part of the present invention.
FIG. 2 is a dot histogram of the observed total ApoCIII abundance from the human plasma samples from patients with normal glucose tolerance (NGT) and patients with impaired glucose tolerance (IGT, pre-diabetes) that were analyzed
   and shown in FIG 1.
FIG. 3 is a dot histogram of the observed total ApoCIII abundance from the human plasma samples from patients with normal glucose tolerance (NGT) and patients with type 2 diabetes (DM) that were analyzed and shown in FIG. 1.
FIG. 4 shows the resultant ROC curves established from the mass spectral total ApoCIII abundance data obtained from each sample population, namely the human plasma samples from patients with normal glucose tolerance (NGT), the human plasma samples from patients with impaired glucose tolerance (IGT, pre-diabetes), and the human plasma samples from patients with type 2 diabetes (DM), that were analyzed and shown in FIG. 1.
FIG. 5 is a dot histogram of the abundance of an ApoCIII variant, namely the observed total ApoCIII(1) abundance, from the human plasma samples from patients with normal glucose tolerance (NGT) and patients with impaired glucose tolerance (IGT, pre-diabetes) that were analyzed and shown in FIG 1.
FIG. 6 shows a dot histogram of the observed total ApoCIII(1) abundance from the human plasma samples from patients with normal glucose tolerance (NGT) and patients with type 2 diabetes (DM) that were analyzed and shown in FIG.1.
FIG. 7 shows the resultant ROC curves established from the mass spectral total ApoCIII(1) abundance data obtained from each sample population, namely the human plasma samples from patients with normal glucose tolerance (NGT), the human plasma samples from patients with impaired glucose tolerance (IGT, pre-diabetes), and the human plasma samples from patients with type 2 diabetes (DM), that were analyzed and shown in FIG. 1.

### DETAILED DESCRIPTION

It is demonstrated for the first time in the present invention that ApoCIII(1) correlates with the clinical diagnosis of both the pre-diabetes and type 2 diabetes disease states. It is also demonstrated for the first time that the modulations in the concentration of ApoCIII(1)compare to the results of the OGTT. This observed phenomenon directly displays the utility of this novel marker as it relates to type 2 diabetes, pre-diabetes, insulin resistance and/or their related conditions or complications.

The present invention encompasses the use of ApoCIII(1)as a biomarker for the in-vitro and/or in-vivo determination/diagnosis/assessment of type 2 diabetes, pre-diabetes, insulin resistance and/or their related conditions or complications. The present invention also includes the use of the ApoCIII(1) variant for the same purpose.

In addition, the present invention includes the use of ApoCIII(1) as a marker for the in-vitro and/or in-vivo screening of compounds, aimed at modulating the expression, activity and/or clearance of ApoCIII(1) for the purpose of treating, or reducing symptoms that are associated with, pre-diabetes, type 2 diabetes, insulin resistance and/or their related conditions or complications. The present invention also includes the use of the ApoCIII(1) variant for this same purpose.
The present invention also includes methodologies for the measurement of ApoCIII(1) from biological samples for the determination/diagnosis/assessment of type 2 diabetes, pre-diabetes, insulin resistance and/ortheir related conditions or complications.
The present invention also extends these methodologies for the measurement of ApoCIII(1) in order to screen compounds, aimed at modulating the expression, activity and/or clearance of ApoCIII(1) for the purpose of treating, or reducing symptoms that are associated with, pre- diabetes, type 2 diabetes, insulin resistance and/ their related conditions or complications.

### Definitions

All the technical and scientific terms used herein, unless defined otherwise, have the meaning commonly understood by a person skilled in art to which this invention pertains. As used herein, the following terms have the meaning ascribed to them unless specified otherwise.

As used herein, "ApoCIII" is a general term describing an apolipoprotein expressed by the gene APOC3. It constitutes 50% of the protein fraction of VLDL and 2% of that of HDL. Its known function is to inhibit lipoprotein lipase and hepatic lipase. It has previously been shown that elevations in ApoCIII levels induce the development of hypertriglyceridemia.

As used herein, "variant" refers to different iso-forms or sub-forms of ApoCIII found in biological samples. Such sub-forms may be the result of truncation of amino acids from the primary structure, alterations or absence of sugars in the associated gylcan, oxidation adducts or expressed genetic mutations.

As used herein, "ApoCIII(0)" refers to the specific ApoCIII variant in which the terminal sialic acid residues on the glycan structure are absent. The ApoCIII(0) variant does not form part of the present invention.
As used herein, "ApoCIII(1)" refers to the specific ApoCIII variant in which there is only a single terminal sialic acid residue on the glycan structure.
As used herein, "ApoCIII(2)" refers to the specific ApoCIII variant in which both the terminal sialic acid residues are present on the glycan structure. The ApoCIII(2) variant does not form part of the present invention.
As used herein, "analysis" refers to the measurement of the activity or concentration of ApoCIII and/or its variants from a biological sample.

As used herein, "biological sample" refers to a fluid or extract having a plurality of components. Complex media may include, but is not limited to, tissue, cell extracts, nuclear extracts, cell lysates and excretions, blood, sera, plasma, saliva, urine, sputum, synovial fluid, cerebral-spinal fluid, tears, feces, saliva, membrane extracts, industrial fluids and the like.

As used herein, "type 2 diabetes (DM)" is a clinical condition defined by excess glucose in the blood. Reference limits that aid in the clinical determination are a Fasting glucose value ≥ 126 mg/dl and/or a 2 hour oral glucose tolerance test value ≥ 200 mg/dl.
As used herein, "pre-diabetes, impaired glucose tolerance (IGT)" is a clinical condition defined by above normal amounts of glucose present in the blood. Reference limits that aid in the clinical determination are Fasting glucose values that are between 110 and 125 mg/dl and/or 2 hour oral glucose tolerance test values that are between 140 and 199 mg/dl.

As used herein, "normal glucose tolerance (NGT)" is a clinical determination when blood glucose levels are within normal concentration ranges. Reference limits that aid in the clinical determination are a Fasting glucose value < 110 mg/dl and/or a 2 hour oral glucose tolerance test value <140 mg/dl.

As used herein, "insulin resistance" is a physical condition in which the hormone insulin becomes less effective in reducing blood glucose levels. The gold standard for evaluating insulin resistance is the administration of a hyperinsulinemic euglycemic clamp.

As used herein, "related conditions or complications" is defined as a variety of ailments that are commonly associated with the development and progression of type 2 diabetes. Some of these ailments include but are not limited to: heart attacks, skin ulcerations, blindness, amputations and kidney failure.

As used herein, "mass spectrometry" refers to the ability to volatilize/ionize analytes to form vapor-phase ions and determine their absolute or relative molecular masses. Suitable forms of volatilization/ionization are laser/light, thermal, electrical, atomized/sprayed and the like or combinations thereof. Suitable forms of mass spectrometry include, but are not limited to, Matrix Assisted Laser Desorption/Time of Flight Mass Spectrometry (MALDI-TOF MS), electrospray (or nanospray) ionization (ESI) mass spectrometry, or the like or combinations thereof.

As used herein, "subject" refers to any human, animal, or other living organism which possesses measurable levels of ApoCIII(1) in a biological sample taken therefrom.
In the present invention, the method for the measurement of activity or concentration of ApoCIII(1)is performed using a biological sample (biological matrix). Possible biological matrices include, but are not limited to, tissue, whole blood, serum, plasma, saliva, cerebral spinal fluid or urine. The sample may also be free or immobilized onto a solid support. Examples of solid supports include, but are not limited to, filter paper, beads, arrays, etc.
Any solid support material known in the art for immobilizing samples may be used.

The sample may also be native in form or have undergone processing including, but not limited to, denaturation, reduction, proteolytic digestion and the like.
In order to measure ApoCIII(1), the present invention may utilize a separation or purification step to isolate or purify the biomarker from the biological sample. This separation can be performed by several means including, but not limited to, chromatography, centrifugation, affinity interactions, chemical methodologies, staining methodologies, and gel electrophoresis. Chromatography techniques may include but are not limited to: high performance liquid chromatography (HPLC), thin layer chromatography (TLC), paper chromatography (PC), affinity chromatography, size exclusion, ion-exchange, reverse phase, etc. Affinity interactions employ the ability of a molecule to bind with another molecule having proper fitting conformation. Affinity methods employed may include, but are not limited to, affinity interactions utilizing antibodies (Ab), antibody fragments (FAb), aptamers, proteins, peptides, lectins, etc. For sandwich assays, combinations of primary and secondary affinity ligands may be used. Gel electrophoresis methods may include, but are not limited to, acrylamide, agarose, etc. Chemical methodologies may include, but are not limited to, amino acid analysis, sequencing, etc. Staining methods may include, but are not limited to, the use of any dye, and the like, that directly binds to the ApoCIII protein and its variants, or to molecules that bind to ApoCIII and its variants.

The method for ApoCIII(1) measurement will utilize a detection technology for the isolated biomarker (i.e. the isolated ApoCIII(1) variant). Detection may be direct or indirect in nature. Detection technologies include but are not limited to: staining, spectrometroscopy, spectrophotometry, colorimetry, fluorescence, luminescence, magnetism, radioisotopes, nuclear magnetic resonance spectroscopy, x-ray crystallography, surface plasmon resonance, mass spectrometry, etc. Depending on the state of the biological sample, the ApoCIII(1) detected may be intact or a fragment thereof.

### Analyses Demonstrating Biomarker Utility

### EXAMPLE

### ANALYSIS OF APOLIPOPROTEIN CIII AND ITS VARIANTS FROM HUMAN PLASMA

One exemplary embodiment of the invention is the concentration measurement of ApoCIII(1) for use as a biomarker(s) to differentiate between clinically defined populations of samples. The analysis of this exemplary embodiment was performed using mass spectrometric immunoassay (MSIA) technology.
In this example, the analyses were performed on human citrate plasma samples obtained from patients that were newly diagnosed and clinically defined as having NGT, IGT or DM. There were 360 plasma samples obtained from NGT individuals, 98 plasma samples from patients with IGT, and 25 plasma samples from patients with DM which were all analyzed in the same manner.
The MSIA was performed with the aid of affinity pipette tips. The affinity pipette tips are made of small, porous microcolumns that are fitted at the entrance of a pipettor tip. The microcolumns are derivatized with an affinity ligand. The affinity ligand used to derivatize the affinity pipettes was anti-ApoCIII antibody.
The sample preparation required the addition of an internal reference standard (IRS) for the semi-quantitative measurement of the ApoCIII. Each sample mixture consisted of 25 µL of ten-fold diluted (with buffer) human plasma, 30 µL of cynomologus monkey plasma (the cynomologus monkey ApoCIII is the IRS) and 145 µL of HEPES buffered saline with 1% tween 20. The prepared samples were interrogated with the anti-ApoCIII affinity pipette tips by repetitive drawing of the samples through the affinity pipettes. The purification process used HEPES buffered saline and water rinses following the ApoCIII affinity retrieval. Enriched and purified proteins were then eluted from the affinity pipettes with sinapic
acid
MALDI matrix and were deposited directly on a MALDI mass spectrometer target for subsequent mass spectral detection.

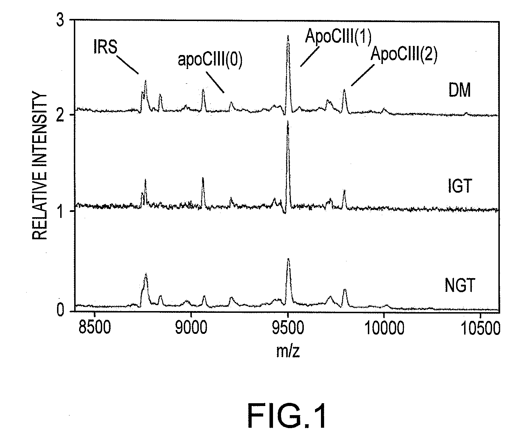

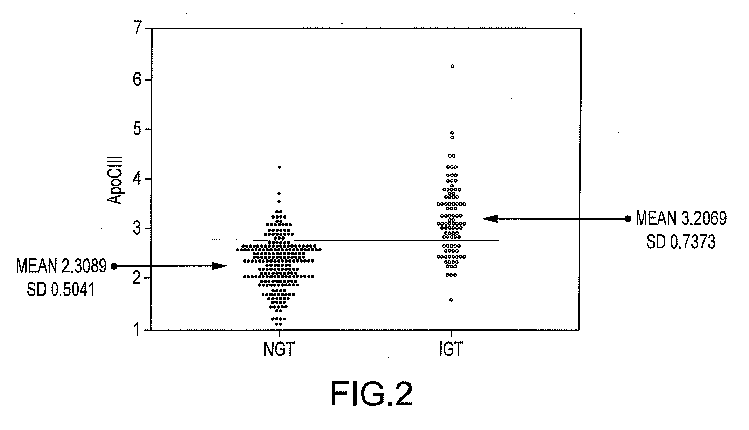

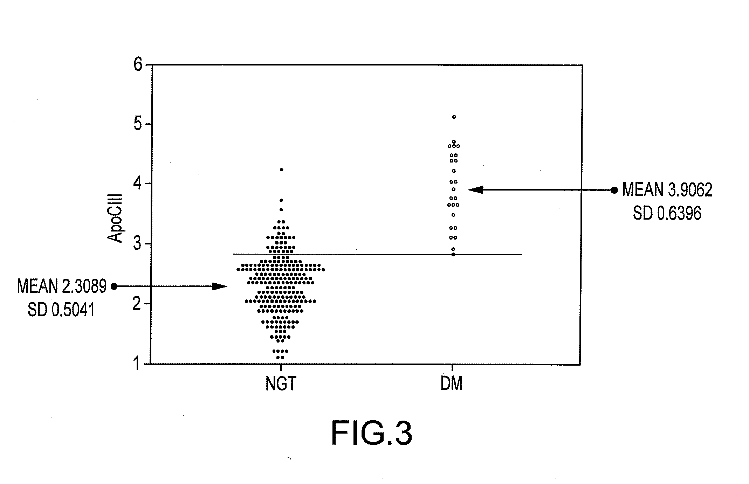

Examination of the resultant mass spectra showed signals from multiple forms of ApoCIII that are present in human plasma, as well as the IRS. Each spectrum was normalized to the integral of the ApoCIII signal obtained from the IRS. Within each mass spectrum, the mass shifted variants of human ApoCIII are clearly separated, with the ApoCIII(1) isoform being the most abundant in the majority of samples. Multiple ApoCIII variants were detected from each sample, but this example focuses on the three most abundant forms; namely ApoCIII(0), ApoCIII(1) and ApoCIII(2). The normalized spectra clearly show that ApoCIII(0), ApoCIII(1), and ApoCIII(2) modulate between the different samples. **FIG. 1** illustrates that the abundance of ApoCIII and its variants is different in human plasma samples from patients with normal glucose tolerance (NGT), impaired glucose tolerance (IGT, pre-diabetes), and type 2 diabetes (DM). FIG. 1 shows that there is an increase in all ApoCIII variants in samples from patients diagnosed with IGT and DM.
The resulting mass spectrometry data showed the ability to discriminate between samples that originate from NGT patients and IGT/DM patients. Using total ApoCIII as a biomarker for IGT, a cut-off value of 2.6090 was established for IGT determination as shown in **FIG. 2****.** The mean value and one standard deviation of each population are also denoted in **FIG. 2****.** Using total ApoCIII as a biomarker for DM, a cut-off value of 2.7890 was established for DM determination as shown in **FIG. 3****.** The mean value and one standard deviation of each population are also denoted in **FIG. 3****.**
The clinical sensitivity and specificity for ApoCIII were also determined. These values are presented below in **Table 1**.

| **Biomarker** | **Sensitivity** | **Specificity** | **Cut-off** |
|---|---|---|---|
| **IGT** | | | |
| ApoCIII | 76.0% | 75.7% | 2.6090 |
| ApoCIII(1) | 82.3% | 83.4% | 1.6600 |

| **DM** | | | |
|---|---|---|---|
| ApoCIII | 100% | 85.5% | 2.7890 |
| ApoCIII(1) | 100% | 94.5% | 1.9140 |

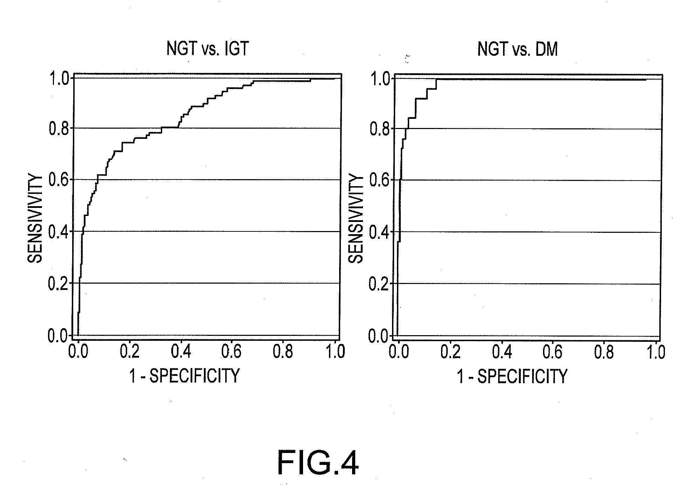

The accuracy of a test to discriminate diseased cases from normal cases is elevated using Receiver Operating Characteristic (ROC) curve analysis. For comparative purposes, the receiver operator characteristic (ROC) curves of the total ApoCIII for IGT and DM were also plotted. These plots are shown in **Fig. 4****.** The displayed plots illustrate the clinical utility of the ApoCIII biomarker in determining/diagnosing both IGT and DM. The calculated areas under the curve for each plot are 0.8492 and 0.9796, respectively.

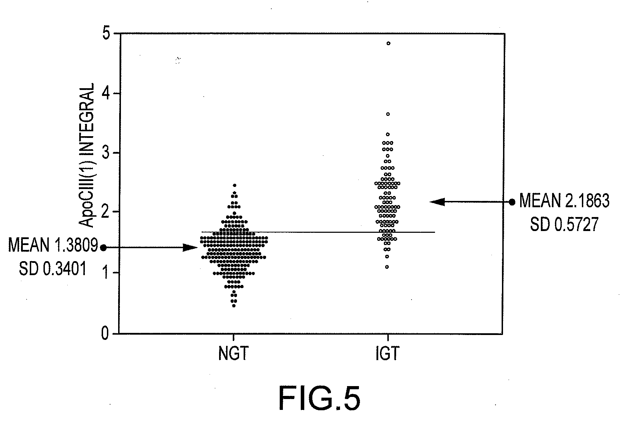

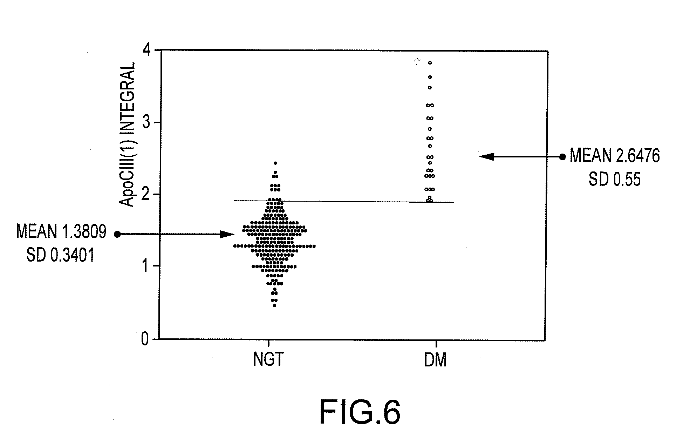

Mass spectrometric immunoassay measurement data of the ApoCIII(1) variant in the samples was then used to perform the same data evaluation. Using ApoCIII(1) as a biomarker for IGT, a cut-off value of 1.6600 was established between the two populations for IGT determination as shown in **FIG. 5****.** The mean value and one standard deviation of each population are also given in **FIG. 5****.** **FIG. 6** shows a similar dot histogram, but instead displays ApoCIII(1) abundance within the NGT and DM samples. Using ApoCIII(1) as a biomarker for DM, a cut-off value of 1.9141 was established to differentiate the two groups for DM determination. The mean value and one standard deviation are also denoted in

### FIG. 6.

The analysis of the ApoCII1(1) variant showed improved clinical utility over the application of the total ApoCIII values. The clinical sensitivity and specificity of the ApoCIII(1) variant as compared to total ApoCIII values is shown in **Table 1** above

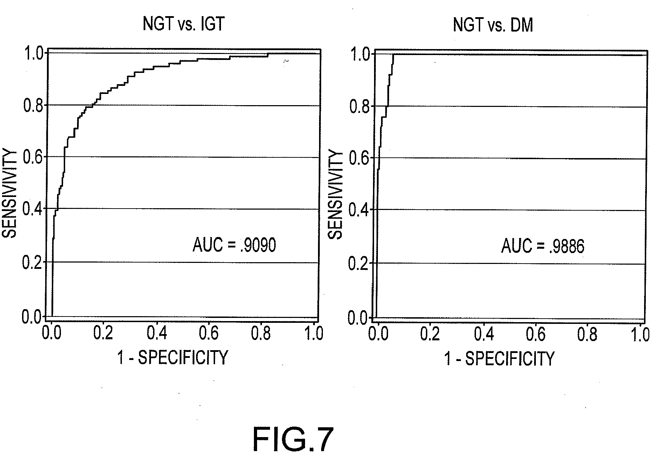

ROC curves for ApoCIII(1) for IGT and DM were also plotted and are shown in **Fig. 7****.** The curves display the clinical utility of the ApoCIII(1) biomarker in determining/diagnosing both IGT and DM. The calculated areas under the curve for each plot are 0.9090 and 0.9886, respectively.
Finally, an algorithm-assisted bio-statistical evaluation of the ApoCIII(1) variant dataset was performed, resulting in improved clinical sensitivity and specificity for determining/diagnosing both IGT and DM. This data is presented below in **Table 2.**

| **Biomarker** | **Sensitivity** | **Specificity** |
|---|---|---|
| **IGT** | | |
| ApoCIII(1) | 93.6% | 84.3% |
| | **DM** | |
| ApoCIII(1) | 98.7% | 95.8% |

All of the analyses set forth and/or described above can also be performed on other ApoCIII variants and/or applied to combinations of various observed ApoCIII variants to determine additional biomarkers or groupings of biomarkers for determining IGT, DM and their related conditions.

Exemplary embodiments of the invention are described above in the Drawings and Description of Exemplary Embodiments. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s). The foregoing description of a preferred embodiment and best mode of the invention known to the applicant at the time of filing the application has been presented and is intended for the purposes of illustration, and description. It is not intended to be exhaustive or to limit the invention to the precise form disclosed, and many modifications and variations are possible in the light of the above teachings within the scope of the appended Claims. Exemplary embodiments were chosen and described in order to best explain the principles of the invention and its practical application and to enable others skilled in the art to
best utilize the invention in various embodiments and with various modifications as are suited to the particular use contemplated.

## Claims

1. A method for diagnosing type 2 diabetes in a biological sample of a subject comprising the steps of detecting a level of ApoCIII(1) in the biological sample of the subject and diagnosing said subject as having diabetes when the level of said ApoCIII(1) in the biological sample of the subject is increased by a statistically significant amount from a level present in a biological sample of one or more subjects with normal glucose tolerance.

2. The method of claim 1 wherein the step of detecting a level of ApoCIII(1) in the biological sample of the subject comprises the step of performing at least one of mass spectroscopy, chromoatography, an affinity reaction, gel electrophoresis, centrifugation, chemical methodology, and staining methodology.

3. The method of claim 1 wherein the step of detecting a level of ApoCIII(1) in the biological sample of the subject comprises the step of performing a single assay.

4. The method of claim 3 further comprising the step of performing a single mass spectrometry.

5. The method of claim 1 wherein the method for diagnosing type 2 diabetes comprises a method for diagnosing pre-diabetes.

6. The method of claim 5 wherein the step of detecting a level of ApoCIII(1) in the biological sample of the subject comprises the step of performing at least one of mass spectroscopy, chromatography, an affinity reaction, gel electrophoresis, centrifugation, chemical methodology, and staining methodology.

7. The method of claim 5 wherein the step of detecting a level of ApoCIII(1) in the biological sample of the subject comprises the step of performing a single assay.

8. The method of claim 7 further comprising the step of performing single mass spectrometry.

9. The method of claim 4 wherein the step of diagnosing said subject as having type 2 diabetes comprises the step of diagnosing said subject as having type 2 diabetes when the level of ApoCIII(1) in the biological sample of the subject has an intensity value of 1.66 or greater.

10. The method of claim 5 wherein the step of diagnosing said subject as having pre-diabetes comprises the step of diagnosing said subject as having pre-diabetes when the level of ApoCIII(1) in the biological sample of the subject has an intensity value of 1.914 or greater.

## Patentansprüche

1. Verfahren zum Diagnostizieren einer Typ-2 Diabetes in einer biologischen Probe eines Subjektes, umfassend die Schritte eines Detektierens eines Maßes an ApoCIII(1) in der biologischen Probe des Subjektes und Stellen einer Diagnose für das Subjekt zum Vorliegen einer Diabetes, wenn das Maß des besagten ApoCIII(1) in der biologischen Probe des Subjektes sich durch einen statistisch signifikanten Betrag von einem Niveau aus erhöht, wie es in einer biologischen Probe von einem oder mehreren Subjekten mit eine normalen Glucose-Toleranz vorliegt.

2. Verfahren nach Anspruch 1, wobei der Schritt eines Detektierens eines Maßes an ApoCIII(1) in der biologischen Probe des Subjektes den Schritt eines Ausführens zumindest entweder einer Massenspektroskopie, einer Chromatografie, einer Affinitätsreaktion, eine Gel-Elektrophorese, einer Zentrifugation, einer chemischen Methodik oder eine Verfärbungs-Methodik umfasst.

3. Verfahren nach Anspruch 1, wobei der Schritt eines Detektierens eines Maßes an ApoCIII(1) in der biologischen Probe des Subjektes den Schritt eines Durchführens eines einzelnen Assays umfasst.

4. Verfahren nach Anspruch 3, ferner aufweisend den Schritt eines Ausführens einer Einzel-Massenspektroskopie.

5. Verfahren nach Anspruch 1, wobei das Verfahren zum Diagnostizieren eines Typ-2 Diabetes ein Verfahren zum Diagnostizieren einer Vordiabetes umfasst.

6. Verfahren nach Anspruch 5, wobei der Schritt eines Detektierens eines Maßes an ApoCIII(1) in der biologischen Probe des Subjektes den Schritt zumindest entweder einer Massenspektroskopie, einer Chromatografie, einer Affinitätsreaktion, eine Gel-Elektrophorese, einer Zentrifugation, einer chemischen Methodik oder eine Verfärbungs-Methodik umfasst.

7. Verfahren nach Anspruch 5, wobei der Schritt eines Detektierens eines Maßes an ApoCIII(1) in der biologischen Probe des Subjektes den Schritt eines Durchführens eines einzelnen Assays umfasst.

8. Verfahren nach Anspruch 7, ferner umfassend den Schritt eines Ausführens einer Einzel-Massenspektroskopie.

9. Verfahren nach Anspruch 4, wobei der Schritt einer Diagnostizierung gegenüber dem Subjekt mit einer Typ-2-Diabetes den Schritt einer Diagnostizierung gegenüber diesem Subjekt als eine Typ-2-Diabetes umfasst, wenn das Maß an ApoCIII(1) in der biologischen Probe des Subjektes einen Intensitätswert von 1,66 oder größer hat.

10. Verfahren nach Anspruch 5, wobei der Schritt einer Diagnostizierung von Vordiabetes gegenüber dem Subjekt den Schritt einer Diagnostizierung gegenüber diesem Subjekt als eine Vordiabetes umfasst wenn das Maß an ApoCIII(1) in der biologischen Probe des Subjektes einen Intensitätswert von 1,914 oder größer hat.

## Revendications

1. Procédé de diagnostic du diabète de type 2 dans un échantillon biologique d'un sujet, comprenant les étapes de détection d'un taux d'ApoCIII(1) dans l'échantillon biologique du sujet et de diagnostic d'un diabète chez ledit sujet lorsque le taux dudit ApoCIII(1) dans l'échantillon biologique du sujet est augmenté d'une quantité statistiquement significative par rapport à un taux présent dans un échantillon biologique d'un ou plusieurs sujets présentant une tolérance au glucose normale.

2. Procédé selon la revendication 1, dans lequel l'étape de détection d'un taux d'ApoCIII(1) dans l'échantillon biologique du sujet comprend l'étape de réalisation d'au moins l'une parmi la spectroscopie de masse, la chromatographie, une réaction d'affinité, une électrophorèse sur gel, une centrifugation, une méthodologie chimique et une méthodologie de coloration.

3. Procédé selon la revendication 1, dans lequel l'étape de détection d'un taux d'ApoCIII(1) dans l'échantillon biologique du sujet comprend l'étape de réalisation d'un dosage unique.

4. Procédé selon la revendication 3, comprenant en outre l'étape de réalisation d'une spectrométrie de masse unique.

5. Procédé selon la revendication 1, dans lequel le procédé de diagnostic du diabète de type 2 comprend un procédé de diagnostic d'un pré-diabète.

6. Procédé selon la revendication 5, dans lequel l'étape de détection d'un taux d'ApoCIII(1) dans l'échantillon biologique du sujet comprend l'étape de réalisation d'au moins l'une parmi la spectroscopie de masse, la chromatographie, une réaction d'affinité, une électrophorèse sur gel, une centrifugation, une méthodologie chimique et une méthodologie de coloration.

7. Procédé selon la revendication 5, dans lequel l'étape de détection d'un taux d'ApoCIII(1) dans l'échantillon biologique du sujet comprend l'étape de réalisation d'un dosage unique.

8. Procédé selon la revendication 7, comprenant en outre l'étape de réalisation d'une spectrométrie de masse unique.

9. Procédé selon la revendication 4, dans lequel l'étape de diagnostic chez ledit sujet d'un diabète de type 2 comprend l'étape de diagnostic chez ledit sujet d'un diabète de type 2 lorsque le taux d'ApoCIII(1) dans l'échantillon biologique du sujet a une valeur d'intensité de 1,66 ou supérieure.

10. Procédé selon la revendication 5, dans lequel l'étape de diagnostic d'un pré-diabète chez ledit sujet comprend l'étape de diagnostic d'un pré-diabète chez ledit sujet lorsque le taux d'ApoCIII(1) dans l'échantillon biologique du sujet a une valeur d'intensité de 1,914 ou supérieure.
